# EUROPEAN PATENT APPLICATION

(11) **EP 4 744 678 A2**
(43) Date of publication of application: **20.05.2026**
(21) Application number: 26153433.3
(22) Date of filing: 18.12.2020
(51) Int. Cl.: A61K 45/06

(54) **LUMATEPERONE FOR USE IN SCHIZOPHRENIA TREATMENT**

(30) Priority: 19.12.2019 US 201962950828 P
(62) Divisional of application: 20900863.0
(71) Applicant: Intra-Cellular Therapies, Inc., Bedminster, NJ 07921 (US)
(72) Inventor: DAVIS, Robert E., New York, 10016 (US); VANOVER, Kimberly, New York, 10016 (US)
(74) Representative: Carpmaels & Ransford LLP

(57) **Abstract**

The present disclosure relates to the treatment of schizophrenia in adults, comprising the administration of pharmaceutical capsules comprising 42 mg of lumateperone (60 mg of lumateperone tosylate).

## Description

### CROSS-REFERENCE TO RELATED APPLICATIONS

This Application is an international application which claims priority to and the benefit of U.S. Provisional Application No. 62/950,828, filed on December 19, 2019, the contents of which are hereby incorporated by reference in its entirety.

### TECHNICAL FIELD

The present disclosure relates to the treatment of schizophrenia in adults, comprising the administration of pharmaceutical capsules comprising 42 mg of lumateperone (60 mg of lumateperone tosylate).

### BACKGROUND

The substituted heterocycle fused gamma-carboline lumateperone, having the chemical name 4-((6b*R*,10a*S*)-3-methyl-2,3,6b,9,10,10a-hexahydro-1*H*,7*H*-pyrido[3',4': 4,5]pyrrolo[1,2,3-*de*]quinoxalin-8-yl)-1-(4-fluoro-phenyl)-butan-1-one, is known to be a serotonin receptor (5-HT_{2A}), dopamine receptor (D₁ and/or D₂), and serotonin transporter (SERT) ligand. It has been disclosed as useful in treating a variety of central nervous system disorders. It exists as a stable, crystalline monotosylate salt, lumateperone tosylate [4-((6b*R*,10a*S*)-3-methyl-2,3,6b,9,10,10a-hexahydro-1*H*,7*H*-pyrido[3',4': 4,5]pyrrolo[1,2,3-de]quinoxalin-8-yl)-1-(4-fluoro-phenyl)-butan-1-one 4-methylbenzenesulfonate].

Lumateperone has been disclosed as an antagonist of the serotonin-2A (5-HT_{2A}) receptor, and/or as a modulator of dopamine receptor signaling at the level of key intra-cellular phosphoproteins. This compound is principally known to be useful for the treatment of positive and negative symptoms of schizophrenia. At dopamine D₂ receptors, this compound has been shown to have dual properties and acts as both a post-synaptic antagonist and a pre-synaptic partial agonist of the D₂ receptor. It also stimulates phosphorylation of glutamatergic NMDA NR2B, or GluN2B, receptors in a mesolimbic specific manner. It is believed that this regional selectivity in the brain areas thought to mediate the efficacy of antipsychotic drugs, together with the serotonergic, glutamatergic, and dopaminergic interactions, may result in antipsychotic efficacy for positive, negative, affective and cognitive symptoms associated with schizophrenia. The compound also exhibits serotonin reuptake inhibition, providing antidepressant activity for the treatment of schizoaffective disorder, and co-morbid depression. Lumateperone displays differential dose-dependent effects, selectively targeting the 5-HT_{2A} receptor at low doses, while progressively interacting with the D₂ receptor at higher doses. As a result, at lower doses, it is useful in treating sleep, aggression and agitation. At a higher dose, it can treat acute exacerbated and residual schizophrenia, bipolar disorders, and mood disorders.

Lumateperone and related compounds have been disclosed in U.S. Pat. No. 6,548,493; 7,238,690; 6,552,017; 6,713,471; U.S. RE39680, and U.S. RE39679, as novel compounds useful for the treatment of disorders associated with 5-HT_{2A} receptor modulation such as anxiety, depression, sleep disorders, and schizophrenia. U.S. 8,598,119, incorporated herein by reference, discloses the use of lumateperone for the treatment of a combination of psychosis and depressive disorders as well as sleep, depressive and/or mood disorders in patients with psychosis. U.S. 8,648,077, incorporated herein by reference, discloses methods of preparing toluenesulfonic acid addition salt crystals of lumateperone. WO2019/178484 discloses that lumateperone may be particularly effective in treating acute depression and acute anxiety owing to its rapid onset of action compared to existing antidepressants. This is believed to be due to signaling through a neurotransmitter system separate from the traditional monoamine signaling systems. Lumateperone provides a dopamine D₁ receptor-dependent enhancement of NMDA and AMPA currents coupled with activation of the mTOR (e.g., mTORC1) signaling pathway.

### BRIEF SUMMARY

The present disclosure provides methods for the treatment of schizophrenia in adults, comprising the administration of pharmaceutical capsules comprising 42 mg of lumateperone (60 mg of lumateperone tosylate) to a patient in need thereof. In some embodiments, the capsules are administered with food. In some embodiments, the patient is a patient without moderate or severe hepatic impairment, or is a patient who is not concomitantly taking a CYP3A4 inducer or CYP3A4 inhibitor, or wherein the patient is not 65 years old or older, or wherein the patient does not have dementia-related psychosis. In some embodiments, the patient has a history of one or more of dementia-related psychosis, seizures, orthostatic hypertension or syncope, cerebrovascular disorder (such as stroke), cardiovascular disorder (such as myocardial infarction or heart failure), tardive dyskinesia, hyperglycemia or diabetes mellitus, dyslipidemia, and difficulty maintaining body weight.

### BRIEF DESCRIPTION OF THE DRAWINGS

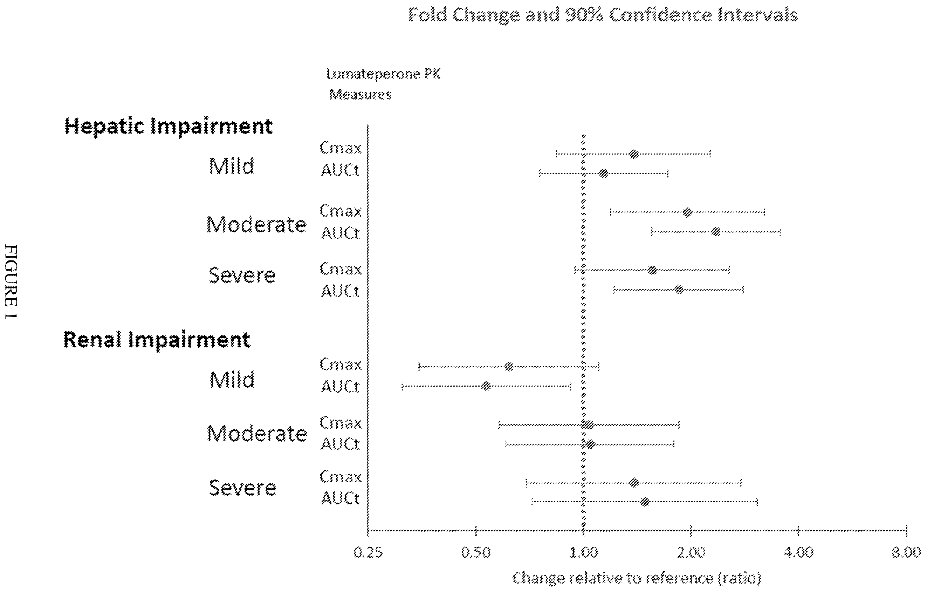

Figure 1. Effects of Intrinsic Factors on Lumateperone Pharmacokinetics. This graph shows the changes (fold change and 90% confidence interval) in Cₘₐₓ (maximum plasma concentration) and AUCt (area under the plasma concentration vs. time curve) in patients with hepatic impairment or renal impairment.

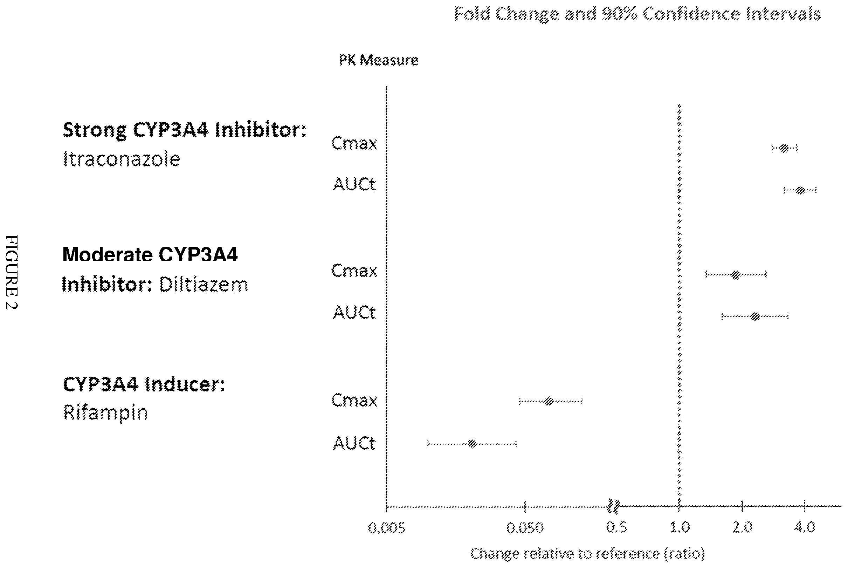

Figure 2. Effects of Other Drugs on Lumateperone Pharmacokinetics. This graph shoes the changes (fold change and 90% confidence interval) in Cₘₐₓ (maximum plasma concentration) and AUCt (area under the plasma concentration vs. time curve) in patients receiving concomitant treatment with itraconazole, diltiazem or rifampin.

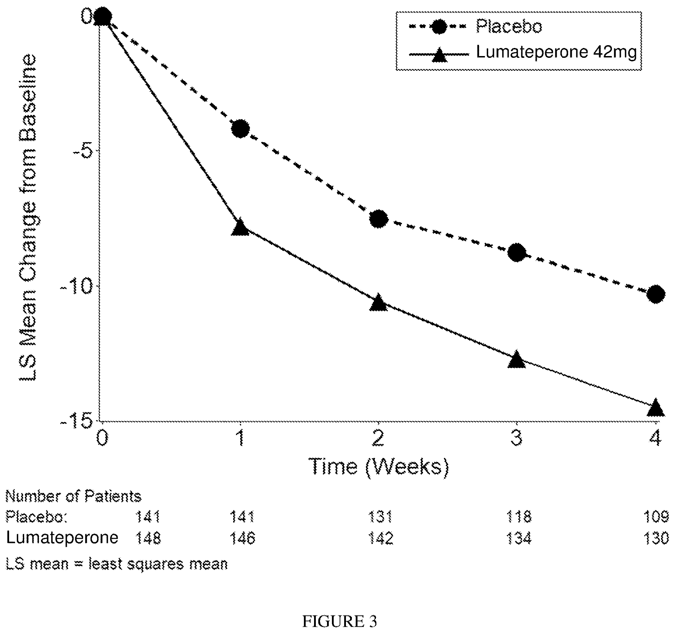

Figure 3. Change from Baseline in PANSS Total Score by Time (Week) in Patients with Schizophrenia in Study 2.

### DETAILED DESCRIPTION

Lumateperone (administered as lumateperone tosylate) is an atypical antipsychotic indicated for the treatment of schizophrenia in adults. Lumateperone is currently has regulatory approval in the United States only for the lumateperone tosylate salt form (lumateperone monotosylate). The recommended dose for this drug is 42 mg of lumateperone administered orally once daily, which corresponds to 60 mg of lumateperone tosylate. Lumateperone is taken orally as a capsule, preferably with food. Unlike other atypical antipsychotics, dose titration is not required.

Therefore, the present disclosure provides a method (Method 1) of treating schizophrenia in an adult patient, wherein the method comprises administering to the patient in need thereof, an oral daily dose of 42 mg lumateperone (e.g., in free or pharmaceutically acceptable salt form), or 60 mg lumateperone tosylate, in the form of capsule. In further embodiments of Method 1, the present disclosure provides:
1.1. Method 1, wherein the oral daily dose of 42 mg lumateperone (60 mg lumateperone tosylate) is provided as a single capsule, optionally wherein the oral daily dose is taken with food.
1.2. Method 1.1, wherein the capsule has a blue cap and opaque white body.
1.3. Method 1.1 or 1.2, wherein the capsule is supplied in a blister pack or in a box (e.g., a box of 30).
1.4. Any of Methods 1.1-1.3, wherein the capsule further comprises croscarmellose sodium, gelatin, magnesium stearate, mannitol, talc, and colorants (e.g., titanium dioxide, FD&C Blue #1, and/or FD&C Red #3).
1.5. Method 1.4, wherein the capsule consists of lumateperone tosylate, croscarmellose sodium, gelatin, magnesium stearate, mannitol, talc, and colorants (e.g., titanium dioxide, FD&C Blue #1, and/or FD&C Red #3).
1.6. Method 1, or any of 1.1-1.5, wherein the patient is less than 65 years of age and/or does not have, or does not have a history of, dementia-related psychosis.
1.7. Method 1, or any of 1.1-1.5, wherein the patient is at least 65 years of age and/or has, or has a history of, dementia-related psychosis.
1.8. Method 1.7, wherein the patient has previously been treated with another atypical antipsychotic but such treatment was terminated due to the development of, or the risk of development of, dementia-related psychosis.
1.9. Method 1, or any of 1.1-1.8, wherein the patient is not under concomitant treatment with a CYP3A4 inducer (e.g., carbamazepine, phenytoin, rifampin, St. John's wort, bosentan, efavirenz, etravirine, modafinil, nafcillin, aprepitant, armodafinil, pioglitazone, prednisone), a moderate or strong CYP3A4 inhibitor (e.g., moderate: amprenavir, ciprofloxacin, cyclosporine, diltiazem, erythromycin, fluconazole, fluvoxamine, verapamil; strong: clarithromycin, grapefruit juice, itraconazole, voriconazole, nefazodone, ritonavir, nelfinavir), and/or a UGT inhibitor (e.g., valproic acid, probenecid).
1.10. Method 1, or any of 1.1-1.8, wherein the patient is under concomitant treatment with a CYP3A4 inducer, a moderate or strong CYP3A4 inhibitor, and/or a UGT inhibitor.
1.11. Method 1.10, wherein the patient has previously been treated with another atypical antipsychotic but such treatment was terminated due to need to undergo concomitant treatment with, or due to side effects resulting from concomitant treatment with, a CYP3A4 inducer, a moderate or strong CYP3A4 inhibitor, and/or a UGT inhibitor.
1.12. Method 1, or any of 1.1-1.11, wherein the patient does not have moderate or severe hepatic impairment (e.g., Child-Pugh Class B or C), or wherein the patient has mild hepatic impairment (e.g., Child-Pugh Class A).
1.13. Method 1, or any of 1.1-1.11, wherein the patient has moderate or severe hepatic impairment (e.g., Child-Pugh Class B or C).
1.14. Method 1.13, wherein the patient has previously been treated with another atypical antipsychotic but such treatment was terminated due to the development of, or the risk of development of, moderate or severe hepatic impairment.
1.15. Method 1, or any of 1.1-1.14, wherein the patient does not have, or is not at risk of developing, tardive dyskinesia.
1.16. Method 1, or any of 1.1-1.14, wherein the patient has, or is at risk of developing, tardive dyskinesia.
1.17. Method 1.16, wherein the patient has previously been treated with another atypical antipsychotic but such treatment was terminated due to the development of, or the risk of development of, tardive dyskinesia.
1.18. Method 1, or any of 1.1-1.17, wherein the patient does not have, or is not at risk of developing, hyperglycemia or diabetes mellitus (e.g., the patient does not have a history of hyperglycemia or diabetes mellitus).
1.19. Method 1, or any of 1.1-1.17, wherein the patient has, or is at risk of developing, hyperglycemia or diabetes mellitus (e.g., the patient has a history of hyperglycemia or diabetes mellitus).
1.20. Method 1.19, wherein the patient has previously been treated with another atypical antipsychotic but such treatment was terminated due to the development of, or the risk of development of, hyperglycemia or diabetes mellitus.
1.21. Method 1, or any of 1.1-1.20, wherein the patient does not have, or is not at risk of developing, dyslipidemia (e.g., the patient does not have a history of dyslipidemia).
1.22. Method 1, or any of 1.1-1.20, wherein the patient has, or is at risk of developing, dyslipidemia (e.g., the patient has a history of dyslipidemia).
1.23. Method 1.22, wherein the patient has previously been treated with another atypical antipsychotic but such treatment was terminated due to the development of, or the risk of development of, dyslipidemia.
1.24. Method 1, or any of 1.1-1.23, wherein the patient does not have, or is not at risk of developing, weight gain (e.g., the patient does not have a history of difficulty controlling weight).
1.25. Method 1, or any of 1.1-1.23, wherein the patient has, or is at risk of developing, weight gain (e.g., the patient has a history of difficulty controlling weight).
1.26. Method 1.25, wherein the patient has previously been treated with another atypical antipsychotic but such treatment was terminated due to the development of, or the risk of development of, weight gain.
1.27. Method 1, or any of 1.1-1.26, wherein the patient does not have, or is not at risk of developing, leukopenia, neutropenia and/or agranulocytosis (e.g., as determined by CBC testing, including WBC and ANC).
1.28. Method 1, or any of 1.1-1.26, wherein the patient has, or is at risk of developing, leukopenia, neutropenia and/or agranulocytosis (e.g., as determined by CBC testing, including WBC and ANC).
1.29. Method 1.28, wherein the patient has previously been treated with another atypical antipsychotic but such treatment was terminated due to the development of, or the risk of development of, leukopenia, neutropenia and/or agranulocytosis (e.g., as determined by CBC testing, including WBC and ANC).
1.30. Method 1, or any of 1.1-1.29, wherein the patient does not have, or is not at risk of developing, orthostatic hypertension or syncope (e.g., the patient does not have a history of orthostatic hypertension or syncope).
1.31. Method 1, or any of 1.1-1.29, wherein the patient has, or is at risk of developing, orthostatic hypertension or syncope (e.g., the patient has a history of orthostatic hypertension or syncope).
1.32. Method 1.31, wherein the patient has previously been treated with another atypical antipsychotic but such treatment was terminated due to the development of, or the risk of development of, orthostatic hypertension or syncope.
1.33. Method 1, or any of 1.1-1.32, wherein the patient does not have, or is not at risk of developing, seizures (e.g., the patient does not have a history of seizures or a lowered seizure threshold).
1.34. Method 1, or any of 1.1-1.32, wherein the patient has, or is at risk of developing, seizures (e.g., the patient has a history of seizures or a lowered seizure threshold).
1.35. Method 1.34, wherein the patient has previously been treated with another atypical antipsychotic but such treatment was terminated due to the development of, or the risk of development of, seizures or a lowered seizure threshold.
1.36. Method 1, or any of 1.1-1.35, wherein the patient does not have, or is not at risk of developing, cognitive or motor impairment (e.g., the patient does not have a history of cognitive or motor impairment).
1.37. Method 1, or any of 1.1-1.35, wherein the patient has, or is at risk of developing, cognitive or motor impairment (e.g., the patient has a history of cognitive or motor impairment).
1.38. Method 1.37, wherein the patient has previously been treated with another atypical antipsychotic but such treatment was terminated due to the development of, or the risk of development of, cognitive or motor impairment.
1.39. Method 1, or any of 1.1-1.38, wherein the patient does not have, or is not at risk of developing, body temperature dysregulation (e.g., the patient does not have a history of body temperature dysregulation, or the patient does not concomitantly engage in strenuous exercise, or is not exposed to extreme heat or dehydration, or does not concomitantly take anticholinergic medications).
1.40. Method 1, or any of 1.1-1.38, wherein the patient has, or is at risk of developing, body temperature dysregulation (e.g., the patient has a history of body temperature dysregulation, or the patient concomitantly engages in strenuous exercise, or is exposed to extreme heat or dehydration, or concomitantly takes anticholinergic medications).
1.41. Method 1.40, wherein the patient has previously been treated with another atypical antipsychotic but such treatment was terminated due to the development of, or the risk of development of, body temperature dysregulation.
1.42. Method 1, or any of 1.1-1.41, wherein the patient is switching from treatment with another atypical antipsychotic to treatment with lumateperone (e.g., lumateperone tosylate), wherein the switch does not include dose titration.
1.43. Any preceding method, wherein the patient has been previously treated with an atypical antipsychotic, such as risperidone, aripiprazole, and olanzapine, and/or quetiapine or ziprasidone.
1.44. Any preceding method, wherein treatment of the patient with an atypical antipsychotic, such as risperidone, aripiprazole, and olanzapine, and/or quetiapine or ziprasidone, is contraindicated.
1.45. Any preceding method, wherein the method does not result in the patient developing tardive dyskinesia.
1.46. Any preceding method, wherein the method does not result in the patient developing hyperglycemia or diabetes mellitus (e.g., as shown by monitoring of fasting glucose levels, insulin levels, and/or hemoglobin A1c levels in plasma).
1.47. Any preceding method, wherein the method does not result in the patient developing dyslipidemia (e.g., as shown by monitoring of plasma lipid panels, including cholesterol, triglycerides, VLDL, LDL and HDL levels).
1.48. Any preceding method, wherein the method does not result in the patient developing weight gain (e.g., as compared to the patient's weight before treatment and/or with reference to the normal body-mass index for a person of said patient's height).
1.49. Any preceding method, wherein the method does not result in the patient developing orthostatic hypertension or syncope.
1.50. Any preceding method, wherein the method further comprises the step of evaluating the patient, prior to the initiation of treatment with lumateperone (e.g., lumateperone tosylate), for signs of cerebrovascular disease, cardiovascular disease, neuroleptic malignant syndrome, tardive dyskinesia, hyperglycemia, diabetes mellitus, dyslipidemia, weight gain, and/or orthostatic vital signs.
1.51. Any preceding method, wherein the method further comprises the step of evaluating the patient, periodically during treatment with lumateperone tosylate, for signs of cerebrovascular disease, cardiovascular disease, neuroleptic malignant syndrome, tardive dyskinesia, hyperglycemia, diabetes mellitus, dyslipidemia, weight gain, and/or orthostatic vital signs (e.g., every 1 month, 2 months, 3 months, 4 months, 6 months, 9 months, 12 months, 18 months or 24 months).
1.52. Any preceding method, wherein the method provides improved sleep (e.g., increase somnolence and/or sedation).
1.53. Any preceding method, wherein the method provides a significant reduction in the PANSS total score within 28 days of the beginning of treatment, e.g., at least a 4-point reduction on the PANSS total score, or at least a 5-point reduction, or at least a 7-point reduction, or at least a 9-point reduction, or at least a 10-point reduction, or at least a 12-point reduction, or at least a 14-point reduction.
1.54. Any preceding method, wherein the method provides a significant reduction in the PANSS total score within 8 days of the beginning of treatment, e.g., at least a 4-point reduction on the PANSS total score, or at least a 5-point reduction, or at least a 6-point reduction, or at least a 7-point reduction, or at least an 8-point reduction.
1.55. Any preceding method, wherein the patient is diagnosed with schizophrenia according to the DSM-5 criteria, or according to DSM-IV-TR criteria.
1.56. Any preceding method, wherein the patient has a baseline PANSS total score of 60 to 100 at the time treatment is initiated, e.g. a PANSS total score of 80 to 90, or 85 to 95, or 88 to 90.

Lumateperone has high binding affinity for serotonin 5-HT_{2A} receptors (Kᵢ = 0.54 nM) and moderate binding affinity for dopamine D₂ (Kᵢ = 32 nM) receptors. Lumateperone has moderate binding affinity for serotonin transporters (Kᵢ = 33 nM). Lumateperone also has moderate binding affinity for dopamine D₁ (Kᵢ = 41 nM) and dopamine D₄ receptors, and for adrenergic alpha_{1A} and alpha_{1B} receptors (Kᵢ projected at < 100 nM), but has low binding affinity (less than 50% inhibition at 100 nM) for muscarinic and histaminergic receptors.

As a such, lumateperone has a very unique receptor binding profile that is not matched by any other existing antipsychotic medications, whether typical or atypical antipsychotics. Nevertheless, lumateperone is currently classified as a member of the atypical antipsychotic class.

The precise mechanism of action of lumateperone in the treatment of schizophrenia is unknown. However, the efficacy of lumateperone could be mediated through a combination of antagonist activity at central serotonin 5-HT_{2A} receptors and postsynaptic antagonist activity at central dopamine D₂ receptors.

Elderly patients with dementia-related psychosis treated with antipsychotic drugs are at an increased risk of death. Analyses of 17 placebo-controlled trials (modal duration of 10 weeks), largely in patients taking atypical antipsychotic drugs, revealed a risk of death in the drug-treated patients of between 1.6 to 1.7 times that in placebo-treated patients. Over the course of a typical 10-week controlled trial, the rate of death in drug-treated patients was about 4.5%, compared to a rate of about 2.6% in placebo-treated patients.

Although the causes of death were varied, most of the deaths appeared to be either cardiovascular (e.g., heart failure, sudden death) or infectious (e.g., pneumonia) in nature.

In placebo-controlled trials in elderly subjects with dementia, patients randomized to risperidone, aripiprazole, and olanzapine had a higher incidence of stroke and transient ischemic attack, including fatal stroke.

It is believed that lumateperone (e.g., lumateperone tosylate) does not present the same risks in elderly patients and patients with dementia-related psychosis that other atypical antipsychotics do. Nevertheless, lumateperone tosylate is not currently approved in the United States for the treatment of patients with dementia-related psychosis.

Neuroleptic Malignant Syndrome (NMS), a potentially fatal symptom complex, has been reported in association with administration of antipsychotic drugs. Clinical manifestations of NMS are hyperpyrexia, muscle rigidity, delirium, and autonomic instability. Additional signs may include elevated creatinine phosphokinase, myoglobinuria (rhabdomyolysis), and acute renal failure. If NMS is suspected in a patient, then such antipsychotic drug should be immediately discontinued, intensive symptomatic treatment and monitoring provided.

It is believed that lumateperone (e.g., lumateperone tosylate) does not present the same risks of NMS as do other atypical antipsychotics.

Tardive dyskinesia, a syndrome consisting of potentially irreversible, involuntary, dyskinetic movements, may develop in patients treated with antipsychotic drugs. The risk appears to be highest among the elderly, especially elderly women, but it is not possible to predict which patients are likely to develop the syndrome. Whether antipsychotic drug products differ in their potential to cause tardive dyskinesia is unknown.

The risk of tardive dyskinesia and the likelihood that it will become irreversible increase with the duration of treatment and the cumulative dose. The syndrome can develop after a relatively brief treatment period, even at low doses. It may also occur after discontinuation of treatment.

Tardive dyskinesia may remit, partially or completely, if antipsychotic treatment is discontinued. Antipsychotic treatment itself, however, may suppress (or partially suppress) the signs and symptoms of the syndrome, possibly masking the underlying process. The effect that symptomatic suppression has upon the long-term course of tardive dyskinesia is unknown.

Antipsychotics are therefore generally prescribed in a manner most likely to reduce the risk of tardive dyskinesia. Chronic antipsychotic treatment should generally be reserved for patients: 1) who suffer from a chronic illness that is known to respond to antipsychotic drugs; and 2) for whom alternative, effective, but potentially less harmful treatments are not available or appropriate. In patients who do require chronic treatment, use the lowest dose and the shortest duration of treatment producing a satisfactory clinical response. The need for continued treatment should be periodically reassessed.

If signs and symptoms of tardive dyskinesia appear in a patient on antipsychotic drug treatment, drug discontinuation should be considered. However, some patients may require treatment with the antipsychotic drug despite the presence of the syndrome.

Antipsychotic drugs have caused metabolic changes, including hyperglycemia, diabetes mellitus, dyslipidemia, and weight gain. Although all of the drugs in the class have been shown to produce some metabolic changes, each drug has its own specific risk profile.

Hyperglycemia, in some cases extreme and associated with ketoacidosis, hyperosmolar coma or death, has been reported in patients treated with antipsychotics. There have been reports of hyperglycemia in patients treated with lumateperone tosylate. Fasting plasma glucose should be assessed before or soon after initiation of antipsychotic medication and monitored periodically during long-term treatment.

In pooled data from short-term (4- to 6-week), placebo-controlled trials of adult patients with schizophrenia, mean changes from baseline and the proportion of patients with shifts from normal to greater than normal levels of fasting glucose in patients treated with lumateperone tosylate were similar to those in patients treated with placebo.

In an uncontrolled open-label trial of lumateperone tosylate for up to 1 year in patients with stable schizophrenia, the percentages of patients with a shift from normal to high in fasting glucose and insulin values were 8% and 12%, respectively. A total of 4.7% of patients with normal hemoglobin A1c (<6.5%) at baseline developed elevated levels (≥6.5%) post-baseline.

Antipsychotics have caused adverse alterations in lipids. Before or soon after initiation of antipsychotic medications, patients should obtain a fasting lipid profile at baseline and this should be monitored periodically during treatment.

In pooled data from short-term (4- to 6-week), placebo-controlled trials of adult patients with schizophrenia, mean changes from baseline and the proportion of patients with shifts to higher levels of fasting total cholesterol and triglycerides were similar in patients treated with lumateperone tosylate and placebo.

In an uncontrolled open-label trial of lumateperone tosylate for up to 1 year in patients with stable schizophrenia, the percentages of patients with a shift from normal to high were 8%, 5%, and 4% for total cholesterol, triglycerides, and LDL cholesterol, respectively.

Weight gain has been observed with use of antipsychotics. Patients should have their weight monitored at baseline and frequently thereafter. In pooled data from placebo-controlled trials of adult patients with schizophrenia, mean changes from baseline and the proportion of subjects with an increase in weight ≥7% from baseline to end of study was similar in patients treated with lumateperone tosylate and placebo.

In an uncontrolled open-label trial of lumateperone tosylate for up to 1 year in patients with stable schizophrenia, the mean change in body weight was approximately -2 kg (SD 5.6) at Day 175 and approximately -3.2 kg (SD 7.4) at Day 350.

Leukopenia and neutropenia have been reported during treatment with antipsychotic agents, including lumateperone tosylate. Agranulocytosis (including fatal cases) have been reported with other agents in the atypical antipsychotic class.

Possible risk factors for leukopenia and neutropenia include pre-existing low white blood cell count (WBC) or absolute neutrophil count (ANC) and history of drug-induced leukopenia or neutropenia. In patients with a pre-existing low WBC or ANC or a history of drug-induced leukopenia or neutropenia, a complete blood count (CBC) should be performed frequently during the first few months of therapy. In such patients, discontinuation of treatment should be considered at the first sign of a clinically significant decline in WBC in the absence of other causative factors.

Patients with clinically significant neutropenia should be monitored for fever or other symptoms or signs of infection and treat promptly if such symptoms or signs occur. Treatment should be discontinued in patients with absolute neutrophil count < 1000/mm3 and WBC values should be followed until recovery.

Atypical antipsychotics cause orthostatic hypotension and syncope. Generally, the risk is greatest during initial dose administration. In clinical trials, the frequencies of orthostatic hypotension for lumateperone tosylate and placebo were 0.7% and 0%, respectively. The rate of syncope for lumateperone tosylate and placebo were 0.2% and 0.2%.

Orthostatic vital signs should be monitored in patients who are vulnerable to hypotension (e.g., elderly patients, patients with dehydration, hypovolemia, and concomitant treatment with antihypertensive medications), patients with known cardiovascular disease (history of myocardial infarction, ischemic heart disease, heart failure, or conduction abnormalities), and patients with cerebrovascular disease. Lumateperone tosylate has not been evaluated in patients with a recent history of myocardial infarction or unstable cardiovascular disease. Such patients were excluded from pre-marketing clinical trials.

Antipsychotics, including lumateperone (e.g., lumateperone tosylate), may cause somnolence, postural hypotension, and motor and sensory instability, which may lead to falls and, consequently, fractures and other injuries. For patients with diseases, conditions or medications that could exacerbate these effects, complete fall risk assessments when initiating antipsychotic treatment and periodically during long-term treatment.

Like other antipsychotic drugs, lumateperone (e.g., lumateperone tosylate), may cause seizures. The risk is greatest in patients with a history of seizures or with conditions that lower the seizure threshold. Conditions that lower the seizure threshold may be more prevalent in older patients.

Lumateperone (e.g., lumateperone tosylate), like other antipsychotics, may cause somnolence and has the potential to impair judgment, thinking, and motor skills. In short-term (i.e., 4- to 6-week) placebo-controlled clinical trials of patients with schizophrenia, somnolence and sedation were reported in 24% of lumateperone tosylate-treated patients, compared to 10% of placebo-treated patients.

Patients taking lumateperone (e.g., lumateperone tosylate) should be cautioned about operating hazardous machinery, including motor vehicles, until they are reasonably certain that therapy with lumateperone tosylate does not affect them adversely.

Atypical antipsychotics may disrupt the body's ability to reduce core body temperature. Strenuous exercise, exposure to extreme heat, dehydration, and anticholinergic medications may contribute to an elevation in core body temperature; lumateperone tosylate should be used with caution in patients who may experience these conditions.

Esophageal dysmotility and aspiration have been associated with antipsychotic drug use. Antipsychotic drugs, including lumateperone (e.g., lumateperone tosylate), should be used cautiously in patients at risk for aspiration.

Concomitant use of lumateperone (e.g., lumateperone tosylate) with moderate or strong CYP3A4 inhibitors increases lumateperone exposure, which may increase the risk of adverse reactions. Such concomitant use should therefore be avoided. Moderate CYP 3A4 inhibitors include amprenavir, ciprofloxacin, cyclosporine, diltiazem, erythromycin, fluconazole, fluvoxamine, and verapamil. Strong CYP 3A4 inhibitors include clarithromycin, grapefruit juice, itraconazole, voriconazole, nefazodone, ritonavir, and nelfinavir.

Concomitant use of lumateperone (e.g., lumateperone tosylate) with CYP3A4 inducers decreases the exposure of lumateperone. Such concomitant use should therefore be avoided. CYP 3A4 inducers include carbamazepine, phenytoin, rifampin, St. John's wort, bosentan, efavirenz, etravirine, modafinil, nafcillin, aprepitant, armodafinil, pioglitazone, and prednisone.

Concomitant use of lumateperone (e.g., lumateperone tosylate) with UGT inhibitors may increase the exposure of lumateperone and/or its metabolites. Such concomitant use should therefore be avoided. UGT inhibitors include valproic acid and probenecid.

Controlled clinical studies of lumateperone tosylate did not include any patients aged 65 or older to determine whether or not they respond differently from younger patients.

Antipsychotic drugs increase the risk of death in elderly patients with dementia-related psychosis. While lumateperone tosylate is not approved for the treatment of patients with dementia-related psychosis, it is believed that lumateperone tosylate does not present the same risks in this regard as other atypical antipsychotics.

Use of lumateperone (e.g., lumateperone tosylate) is not recommended for patients with moderate (Child-Pugh class B) to severe hepatic impairment (Child-Pugh class C). Patients with moderate and severe hepatic impairment have experienced higher exposure to lumateperone. No dosage adjustment is recommended for patients with mild hepatic impairment (Child-Pugh A).

The words "treatment" and "treating" are to be understood accordingly as embracing prophylaxis and treatment or amelioration of symptoms of disease and/or treatment of the cause of the disease. In particular embodiments, the words "treatment" and "treating" refer to prophylaxis or amelioration of symptoms of the disease.

The term "patient" means a human patient.

The term "adult" means a patient of at least 18 years of age.

The term "lumateperone" as used herein without further specificity is understood to embrace lumateperone free base and all pharmaceutically acceptable salts of lumateperone, including, but not limited to, lumateperone tosylate, lumateperone besylate, lumateperone hydrochloride, lumateperone oxalate, lumateperone aminosalicylate, lumateperone cyclamate, lumateperone nosylate, and lumateperone mesylate. In some embodiments, said lumateperone in free base or pharmaceutically acceptable salt form is in a crystalline form.

As used herein, unless specified otherwise, the term "lumateperone tosylate" refers to either lumateperone monotosylate, lumateperone bistosylate, or any other acid addition salt form of lumateperone comprising toluenesulfonic acid (including salts having a base to acid ratio of less than 1:1 and mixed salts comprising other acid anions). In some embodiments, any reference herein to "lumateperone tosylate" may refer to lumateperone monotosylate. Currently, only lumateperone monotosylate has been studied in human clinical trials and given regulatory approval for human administration. Thus, references to "lumateperone tosylate" with respect clinical trial results and regulatory approval herein is understood to refer to lumateperone monotosylate.

As used herein, references to patients "at risk of" having or developing any side effects or disorders may refer to such patients having any family history of said side effects or disorders, or may refer such patients having symptoms or clinical test results consistent with the risk of having or developing said side effects or disorders. The evaluation of such risks is within the skill of the generalist or specialist physician. For example, a patient at risk of having or developing diabetes may be evaluated based on fasting blood glucose level, fasting blood insulin level, glucose tolerance test, serum hemoglobin A1c, family history of diabetes, other risk factors for diabetes (such as body weight, cardiovascular health). It is understood that for several of these factors, test result may indicate either the presence of diabetes or a heightened risk for developing diabetes. Similarly, a patient having or at risk of having dyslipidemia may be evaluated based on fasting blood lipid levels (e.g., cholesterol; HDL, LDL and VLDL lipoproteins; lipoprotein ratios; triglycerides), past history of elevated blood lipid levels, and family history of dyslipidemia or heart disease (e.g., ischemic heart disease, coronary artery disease), or genetic variations associated with lipid disease. Similarly, a patient having or at risk of having hepatic impairment (or gradations in hepatic impairment) may be evaluated based on blood or hepatic biopsy levels of hepatic enzymes (e.g., ALT, AST, alkaline phosphatase) or evidence of current or past hepatitis (e.g., circulating antibodies to hepatitis A, B or C antigens), known risk factors for hepatic impairment (e.g., chronic alcoholism), or evidence of early-stage hepatic disease (e.g., fatty liver disease). Similarly, a patient having or at risk of having cognitive or motor impairment may be evaluated based on a patient's clinical examination (including memory tests, and diagnostic imaging such as CT and MRI, history of seizures), family history or diagnosis of diseases such as Alzheimer's disease and Parkinson's disease, and history of trauma (such as acute head trauma or evidence of traumatic brain injury).

References to "weight gain" herein may refer to changes in weight of a patient and/or to a comparison of a patient's body mass index (BMI) to normalized BMI ranges for said patient's height.

Methods of synthesizing lumateperone and related compounds are known in art, and include the methods disclosed in in U.S. Patents Nos. 6,548,493; 7,238,690; 6,552,017; 6,713,471; 7,183,282; 7,081,455; 8,309,722; U.S. RE39680, and U.S. RE39679, and US 2017/183350, the contents of each of which are incorporated by reference in their entirety. Salts of the Compounds of the Invention may also be prepared as similarly described in U.S. Patent Nos. 6,548,493; 7,238,690; 6,552,017; 6,713,471; 7,183,282; 8,648,077; U.S. RE39680; U.S. RE39679; the contents of each of which are incorporated by reference in their entirety.

### Example 1: Clinical Trials Experience

Because clinical trials are conducted under widely varying conditions, adverse reaction rates observed in the clinical trials of a drug cannot be directly compared to rates in the clinical trials of another drug and may not reflect the rates observed in practice.

The safety of lumateperone tosylate (lumateperone monotosylate specifically) has been evaluated in 1724 adult patients with schizophrenia exposed to one or more doses. Of these patients, 811 participated in short-term (4- to 6-week), placebo-controlled trials with doses ranging from 14 to 84 mg/day. A total of 329 lumateperone tosylate-exposed patients had at least 6 months of exposure and 108 had at least 1 year of exposure to the 60-mg dose of lumateperone tosylate.

There was no single adverse reaction leading to discontinuation that occurred at a rate of >2% in lumateperone tosylate -treated patients.

The most common adverse reactions (incidence of at least 5% of subjects exposed to lumateperone tosylate and greater than twice the rate of placebo) were somnolence/sedation and dry mouth.

Adverse reactions associated with lumateperone tosylate (incidence of at least 2% in patients exposed to lumateperone tosylate and greater than placebo) are shown in the table below. The following findings are based on the pooled short-term (4- to 6-week), placebo-controlled studies in adult patients with schizophrenia in which lumateperone tosylate was administered at a daily dose of 42 mg (N=406).

| Adverse Reactions Reported in ≥ 2% of Lumateperone Tosylate-Treated Patients in 4- to 6-Week | Placebo | Lumateperone tosylate (60 mg) |
|---|---|---|
| | (N=412) | |
| Schizophrenia Trials | | (N=406) |
| Somnolence/ Sedation | 10% | 24% |
| Nausea | 5% | 9% |
| Dry Mouth | 2% | 6% |
| Dizziness (dizziness, dizziness postural) | 3% | 5% |
| Creatine Phosphokinase Increased | 1% | 4% |
| Fatigue | 1% | 3% |
| Vomiting | 2% | 3% |
| Hepatic Enzymes Increased (ALT, AST, "hepatic enzymes" increased, or liver function tests abnormal | 1% | 2% |

Dystonia: Symptoms of dystonia, prolonged abnormal contractions of muscle groups, may occur in susceptible individuals during the first few days of treatment. Dystonic symptoms include: spasm of the neck muscles, sometimes progressing to tightness of the throat, swallowing difficulty, difficulty breathing, and/or protrusion of the tongue. Although these symptoms can occur at low doses, they occur more frequently and with greater severity with high potency and higher doses of first-generation antipsychotic drugs. An elevated risk of acute dystonia is observed in males and younger age groups.

Extrapyramidal Symptoms: In the 4- to 6-week, placebo-controlled studies, the frequency of reported events related to extrapyramidal symptoms (EPS), including akathisia, extrapyramidal disorder, muscle spasms, restlessness, musculoskeletal stiffness, dyskinesia, dystonia, muscle twitching, tardive dyskinesia, tremor, drooling, and involuntary muscle contractions was 6.7% for lumateperone tosylate and 6.3% for placebo.

In the 4- to 6-week trials, data were collected using the Simpson Angus Scale (SAS) for EPS (total score ranges from 0 to 40), the Barnes Akathisia Rating Scale (BARS) for akathisia (total score ranges from 0 to 14), and the Abnormal Involuntary Movement Scale (AIMS) for dyskinesia (total score ranges from 0 to 28). The mean changes from baseline for lumateperone tosylate-treated patients and placebo-treated patients were 0.1 and 0 for the SAS, -0.1 and 0 for the BARS, and 0.1 and 0 for the AIMS, respectively.

QTcF interval was evaluated in a randomized, placebo- and active- (moxifloxacin 400 mg) controlled, four-arm crossover study utilizing concentration-QTc effect modeling in 33 patients with schizophrenia. The placebo-corrected change from baseline QTcF (90% two-sided upper confidence interval) values of 4.9 (8.9) and 15.8 (19.8) ms for the 42 mg and the supratherapeutic dose of 126 mg (three times the recommended daily dosage) lumateperone tosylate, respectively, administered orally once daily for 5 days.

Specifically, lumateperone tosylate was evaluated for the treatment of schizophrenia in two placebo-controlled trials.

Study 1 (NCT01499563) was a four-week, randomized, double-blind, placebo-controlled, multi-center study in adult patients with a diagnosis of schizophrenia according to the DSM-IV-TR criteria. The primary efficacy measure was the Positive and Negative Syndrome Scale (PANSS) total score. The PANSS is a 30-item scale used to measure symptoms of schizophrenia. Each item is rated by a clinician on a seven-point scale. A score of 1 indicates the absence of symptoms, and a score of 7 indicates extremely severe symptoms. The PANSS total score may range from 30 to 210 with higher scores reflecting greater overall symptom severity.

A total of 335 patients were randomized to receive lumateperone tosylate 42 mg, lumateperone tosylate 84 mg (two times the recommended daily dose), an active comparator, or placebo. The study was not designed to allow for efficacy comparison of lumateperone tosylate and the active comparator. Demographic and baseline disease characteristics were similar for the lumateperone tosylate, active comparator, and placebo groups. Median age was 42 years (range 20 to 55 years). 17% were female, 19% were Caucasian, and 78% were African American.

Compared to the placebo group, patients randomized to lumateperone tosylate 42 mg showed a statistically significant reduction from baseline to Day 28 in the PANSS total score. The treatment effect in the lumateperone tosylate 84 mg group (vs. placebo) was not statistically significant.

Study 2 (NCT02282761) was a four-week, randomized, double-blind, placebo-controlled, multi-center study in adult patients with a diagnosis of schizophrenia according to the DSM-5 criteria. The primary efficacy measure was the PANSS total score.

A total of 450 patients were randomized to receive lumateperone tosylate 28 mg (two-thirds of the recommended daily dose), lumateperone tosylate 42 mg, or placebo. Demographic and baseline disease characteristics were similar for the lumateperone tosylate and placebo groups. Median age was 44 years (range 19 to 60 years); 23% were female, 26% were Caucasian and 66% were African American.

Compared to the placebo group, patients randomized to lumateperone tosylate 42 mg showed a statistically significant reduction from baseline to Day 28 in the PANSS total score. The treatment effect in the lumateperone tosylate 28 mg group (vs. placebo) was not statistically significant.

The results of Study 1 and Study 2 are shown in the table below:

| | | | Primary Efficacy Endpoint: PANSS Total | | |
|---|---|---|---|---|---|
| Study Number | Treatment Group | N | Mean Baseline Score (SD) | LS Mean Change from Baseline (SE) | Placebo-subtracted Difference |
| | | | | | (95% CI)^{a} |
| 1 | Lumateperone tosylate (42 mg)* | 84 | 88.1 (11.0) | -13.2 (1.7) | -5.8 (10.5, -1.1) |
| | Placebo | 85 | 86.3 (13.1) | -7.4 (1.7) | -- |
| 2 | Lumateperone tosylate (42 mg)* | 150 | 90.0 (9.6) | -14.5 (1.3) | -4.2 (-7.8, -0.6) |
| | Placebo | 150 | 89.0 (10.3) | -10.3 (1.3) | -- |

The PANSS total score may range from 30 to 210; higher scores reflect greater symptom severity. SD: standard deviation; SE: standard error; LS Mean: least-squares mean; CI: unadjusted confidence interval. ^{a} Difference (drug minus placebo) in LS mean change from baseline not adjusted for sample size increase after unblinded interim analysis. * Statistically significantly superior to placebo.

Studies 1 and 2 did not include any patients aged 65 or older. Examination of subgroups by race and sex did not suggest differences in response in either study.

The change from baseline in PANSS total score by time in Patients with Schizophrenia in Study 2 is shown in Figure 3.

### Example 2: Pharmacokinetic Studies

Following once daily oral administration of lumateperone tosylate, lumateperone steady state is reached in about 5 days. Increase in steady-state exposure is approximately dose-proportional in the range of 21 mg to 56 mg. A large inter-subject variability in lumateperone PK parameters was observed, with coefficients of variation for Cₘₐₓ (peak plasma concentration) and AUC (area under the concentration vs time curve) ranging from 68% to 97% at steady state.

Absorption: The absolute bioavailability of lumateperone capsules is about 4.4%. Cₘₐₓ of lumateperone is reached approximately 1-2 hours after lumateperone tosylate dosing.

Food Effect: Ingestion of a high-fat meal with lumateperone tosylate lowers lumateperone mean Cₘₐₓ by 33% and increases mean AUC by 9%. Median Tₘₐₓ was delayed about 1 hour (from 1 hour at fasted state to 2 hours in the presence of food).

Distribution: Protein binding of lumateperone is 97.4% at 5 µM (about 70-fold higher than therapeutic concentrations) in human plasma. The volume of distribution of lumateperone following intravenous administration is about 4.1 L/kg.

Elimination: The clearance of lumateperone is approximately 27.9 L/hour and the terminal half-life is about 18 hours after intravenous administration.

Metabolism: Lumateperone is extensively metabolized with more than twenty metabolites identified in vivo. After a single ¹⁴C-labeled oral dose, lumateperone and glucuronidated metabolites represent about 2.8% and 51% of the total plasma radioactivity, respectively. *In vitro* studies show that multiple enzymes, including but not limited to, uridine 5'-diphospho-glucuronosyltransferases (UDP-glucuronosyltransferase, UGT) 1A1, 1A4, and 2B15, aldoketoreductase (AKR) 1C1, 1B10, and 1C4, and cytochrome P450 (CYP) 3A4, 2C8, and 1A2, are involved in the metabolism of lumateperone.

Excretion: In a human mass balance study, 58% and 29% of the radioactive dose was recovered in the urine and feces, respectively. Less than 1% of the dose was excreted as unchanged lumateperone in the urine.

Specific Populations: Effects of hepatic or renal impairment on lumateperone exposure are presented in Figure 1. No clinically significant differences in the pharmacokinetics of lumateperone were observed based on age, sex, or race.

Drug Interaction Studies: The effects of other drugs on the exposures of lumateperone are presented in Figure 2. CYP3A4 substrates: No clinically significant differences in the pharmacokinetics of midazolam (CYP3A4 substrate) or its metabolite 1-hydroxymidazolam were observed when used concomitantly with single or multiple doses of lumateperone in patients with schizophrenia.

*In Vitro* Studies: Lumateperone showed little to no inhibition of CYP1A2, CYP2C9, CYP2C19, CYP2D6, or CYP3A4/5. It showed no induction of CYP1A2, CYP2B6, or CYP3A4. Lumateperone did not appear to be a P-gp or BCRP substrate. It showed little to no inhibition of OCT2, OAT1, OAT3, OATP1B3, or OATP1B1.

### NUMBERED EMBODIMENTS

1. A method of treating schizophrenia in an adult patient, wherein the method comprises administering to the patient in need thereof, an oral daily dose of 42 mg lumateperone, or 60 mg lumateperone tosylate, in the form of capsule, optionally wherein the oral daily dose is taken with food.
2. The method according to embodiment 1, wherein the oral daily dose of 42 mg lumateperone (60 mg lumateperone tosylate) is provided as a single capsule.
3. The method according to embodiment 1 or 2, wherein the capsule further comprises croscarmellose sodium, gelatin, magnesium stearate, mannitol, talc, and colorants (e.g., titanium dioxide, FD&C Blue #1, and/or FD&C Red #3).
4. The method according to any one of embodiments 1-3, wherein the patient is less than 65 years of age and/or does not have, or does not have a history of, dementia-related psychosis.
5. The method according to any one of embodiments 1-4, wherein the patient is not under concomitant treatment with a CYP3A4 inducer (e.g., carbamazepine, phenytoin, rifampin, St. John's wort, bosentan, efavirenz, etravirine, modafinil, nafcillin, aprepitant, armodafinil, pioglitazone, prednisone), a moderate or strong CYP3A4 inhibitor (e.g., moderate: amprenavir, ciprofloxacin, cyclosporine, diltiazem, erythromycin, fluconazole, fluvoxamine, verapamil; strong: clarithromycin, grapefruit juice, itraconazole, voriconazole, nefazodone, ritonavir, nelfinavir), and/or a UGT inhibitor (e.g., valproic acid, probenecid).
6. The method according to any one of embodiments 1-5, wherein the patient does not have moderate or severe hepatic impairment (e.g., Child-Pugh Class B or C), or wherein the patient has mild hepatic impairment (e.g., Child-Pugh Class A).
7. The method according to any one of embodiments 1-6, wherein the patient has, or is at risk of developing, hyperglycemia or diabetes mellitus (e.g., the patient has a history of hyperglycemia or diabetes mellitus).
8. The method according to any one of embodiments 1-6, wherein the patient has previously been treated with another atypical antipsychotic but such treatment was terminated due to the development of, or the risk of development of, hyperglycemia or diabetes mellitus.
9. The method according to any one of embodiments 1-8, wherein the patient has, or is at risk of developing, weight gain (e.g., the patient has a history of difficulty controlling weight).
10. The method according to any one of embodiments 1-9, wherein the patient has previously been treated with another atypical antipsychotic but such treatment was terminated due to the development of, or the risk of development of, weight gain.
11. The method according to any one of embodiments 1-10, wherein the patient is switching from treatment with another atypical antipsychotic to treatment with lumateperone tosylate, wherein the switch does not include dose titration.
12. The method according to any one of embodiments 1-11, wherein the patient has been previously treated with an atypical antipsychotic, such as risperidone, aripiprazole, and olanzapine
13. The method according to any one of embodiments 1-12, wherein treatment of the patient with an atypical antipsychotic, such as risperidone, aripiprazole, and olanzapine, is contraindicated.
14. The method according to any one of embodiments 1-13, wherein the method does not result in the patient developing weight gain.
15. The method according to any one of embodiments 1-14, wherein the method provides a significant reduction in the PANSS total score within 28 days of the beginning of treatment, e.g., at least a 4-point reduction on the PANSS total score, or at least a 5-point reduction, or at least a 7-point reduction, or at least a 9-point reduction, or at least a 10-point reduction, or at least a 12-point reduction, or at least a 14-point reduction.
16. The method according to any one of embodiments 1-15, wherein the method provides a significant reduction in the PANSS total score within 8 days of the beginning of treatment, e.g., at least a 4-point reduction on the PANSS total score, or at least a 5-point reduction, or at least a 6-point reduction, or at least a 7-point reduction, or at least an 8-point reduction.
17. The method according to any one of embodiments 1-16, wherein the patient has a baseline PANSS total score of 60 to 100 at the time treatment is initiated, e.g. a PANSS total score of 80 to 90, or 85 to 95, or 88 to 90.

## Claims

1. Lumateperone for use in a method of treating schizophrenia in an adult patient, wherein the method comprises administering to the patient in need thereof, an oral daily dose of 42 mg lumateperone, or 60 mg lumateperone tosylate, in the form of capsule, wherein the patient is switching from treatment with another atypical antipsychotic to treatment with said lumateperone or lumateperone tosylate, wherein the switch does not include dose titration.

2. Lumateperone for use according to claim 1, wherein the previous atypical antipsychotic was risperidone, aripiprazole, olanzapine, quetiapine, or ziprasidone.

3. Lumateperone for use according to claim 1 or 2, wherein the patient has moderate or severe hepatic impairment (e.g., Child-Pugh Class B or C).

4. Lumateperone for use according to any one of claims 1-3, wherein use of the previous atypical antipsychotic was terminated due to the development of, or the risk of development of, moderate or severe hepatic impairment.

5. Lumateperone for use according to any one of claims 1-4, wherein use of the previous atypical antipsychotic was terminated due to need to undergo concomitant treatment with, or due to side effects resulting from concomitant treatment with, a CYP3A4 inducer, a moderate or strong CYP3A4 inhibitor, and/or a UGT inhibitor.

6. Lumateperone for use according to any one of claims 1-5, wherein the patient has, or is at risk of developing, hyperglycemia or diabetes mellitus (e.g., the patient has a history of hyperglycemia or diabetes mellitus).

7. Lumateperone for use according to any one of claims 1-6, wherein use of the previous atypical antipsychotic was terminated due to the development of, or the risk of development of, hyperglycemia or diabetes mellitus.

8. Lumateperone for use according to any one of claims 1-7, wherein the patient has, or is at risk of developing, weight gain (e.g., the patient has a history of difficulty controlling weight).

9. Lumateperone for use according to any one of claims 1-8, wherein use of the previous atypical antipsychotic was terminated due to the development of, or the risk of development of, weight gain.

10. Lumateperone for use according to any one of claims 1-9, wherein the patient has, or is at risk of developing, leukopenia, neutropenia and/or agranulocytosis (e.g., as determined by CBC testing, including WBC and ANC).

11. Lumateperone for use according to any one of claims 1-10, wherein use of the previous atypical antipsychotic was terminated due to the development of, or the risk of development of, leukopenia, neutropenia and/or agranulocytosis (e.g., as determined by CBC testing, including WBC and ANC).

12. Lumateperone for use according to any one of claims 1-11, wherein the oral daily dose is taken with food.

13. Lumateperone for use according to any one of claims 1-12, wherein the oral daily dose of 42 mg lumateperone (60 mg lumateperone tosylate) is provided as a single capsule, optionally wherein the capsule further comprises croscarmellose sodium, gelatin, magnesium stearate, mannitol, talc, and colorants (e.g., titanium dioxide, FD&C Blue #1, and/or FD&C Red #3).

14. Lumateperone for use according to any one of claims 1-13, wherein the patient is not under concomitant treatment with a CYP3A4 inducer (e.g., carbamazepine, phenytoin, rifampin, St. John's wort, bosentan, efavirenz, etravirine, modafinil, nafcillin, aprepitant, armodafinil, pioglitazone, prednisone), a moderate or strong CYP3A4 inhibitor (e.g., moderate: amprenavir, ciprofloxacin, cyclosporine, diltiazem, erythromycin, fluconazole, fluvoxamine, verapamil; strong: clarithromycin, grapefruit juice, itraconazole, voriconazole, nefazodone, ritonavir, nelfinavir), and/or a UGT inhibitor (e.g., valproic acid, probenecid).

15. Lumateperone for use according to any one of claims 1-14, wherein the patient does not have moderate or severe hepatic impairment (e.g., Child-Pugh Class B or C), or wherein the patient has mild hepatic impairment (e.g., Child-Pugh Class A).

16. Lumateperone for use according to any one of claims 1-15, wherein the method does not result in the patient developing weight gain.

17. Lumateperone for use according to any one of claims 1-16, wherein the method provides a significant reduction in the PANSS total score within 28 days of the beginning of treatment, e.g., at least a 4-point reduction on the PANSS total score, or at least a 5-point reduction, or at least a 7-point reduction, or at least a 9-point reduction, or at least a 10-point reduction, or at least a 12-point reduction, or at least a 14-point reduction.

18. Lumateperone for use according to any one of claims 1-17, wherein the method provides a significant reduction in the PANSS total score within 8 days of the beginning of treatment, e.g., at least a 4-point reduction on the PANSS total score, or at least a 5-point reduction, or at least a 6-point reduction, or at least a 7-point reduction, or at least an 8-point reduction.

19. Lumateperone for use according to any one of claims 1-18, wherein the patient has a baseline PANSS total score of 60 to 100 at the time treatment is initiated, e.g. a PANSS total score of 80 to 90, or 85 to 95, or 88 to 90.

20. Lumateperone for use according to any one of claims 1-19, wherein the patient is diagnosed with schizophrenia according to the DSM-5 criteria, or according to DSM-IV-TR criteria.
